# EUROPEAN PATENT SPECIFICATION

(11) **EP 1 481 640 B1**
(45) Date of publication and mention of the grant of the patent: **10.10.2007**
(21) Application number: 04252977.6
(22) Date of filing: 20.05.2004
(51) Int. Cl.: A61B 17/122

(54) **Surgical clip**
Chirurgische Klammer
Clip chirurgical

(30) Priority: 28.05.2003 US 446800
(43) Date of publication of application: 01.12.2004
(73) Proprietor: ETHICON ENDO-SURGERY, INC., Cincinnati, Ohio 45242 (US)
(72) Inventor: Huitema, Thomas W., Cincinnati, Ohio 45240 (US)
(74) Representative: Tunstall, Christopher Stephen

(56) References cited:
- US-A- 4 805 618
- US-A1- 2002 010 481
- US-A1- 2002 072 759
- US-A1- 2002 111 641

## Description

### Field of the Invention

The present invention has application in conventional endoscopic and open surgical instrumentation as well application in robotic-assisted surgery. The present invention has even further relation to surgical clips.

### Background of the Invention

In recent years, there have been many advances in endoscopic and laparoscopic surgical procedures. In these procedures, a surgeon makes an incision at the desired location where the surgical procedure is to be performed. Typically, a trocar is then inserted into the incision made by the surgeon. By applying pressure against the proximal end of the trocar, the obturator is forced through the tissue until it enters a target location, such as the abdominal cavity or any other desired hollow viscus of the body. The cannula is inserted through the perforation made by the obturator and the obturator is withdrawn, leaving the cannula as an accessway to the abdominal cavity. If desired, a pressurizing gas such as, for example, carbon dioxide can be pumped through the cannula of the trocar to inflate the abdomen or hollow viscus of the body. Then, any number of surgical instruments such as, for example, a tissue fastening instrument can be inserted through the cannula of the trocar to perform the surgical procedure.

One such tissue fastening instrument inserted through the cannula during a surgical procedure is the clip applier. Clip appliers are employed by the surgeon during the procedure to sequentially or simultaneously apply one or more clips to body tissue for the purpose of pinching vessels. An example of a clip applier is disclosed in US Patent No. 5,843,097 issued to Mayenberger et al. A surgical applicator for U-shaped clips is described comprising a handle, a tubular shaft adjoining the handle, a forceps-type applicator tool at the free end of the tubular shaft, a clip magazine in the tubular shaft, a closing mechanism comprising of jaws at the distal end of the tubular shaft, and an advancing mechanism arranged in the tubular shaft. The advancing mechanism pushes a clip into the jaws of the closing mechanism. When the handle is actuated, the jaws of the closing mechanism pinch the clip around the vessel.

Unfortunately, during the surgical procedure, the surgeon sometimes positions the cannula slightly away from the vessel to be pinched. Therefore, articulating surgical instruments have been developed to allow rotation of the end effector to reach vessels positioned away from the cannula. One example of an articulating surgical instrument is disclosed in US Patent No. 5,702,408 issued to Wales et al. A four-bar linkage for an articulation assembly adapted for use with a surgical instrument is disclosed. The first link is in the form of an actuation lever which pivots transversely to the longitudinal axis of the instrument. The second and third links may be operatively connected to the first link. The fourth link is in the form of an end effector for the instrument, and it is rotatably attached to the second and third links for movement transverse to the longitudinal axis. When the first link is pivotally rotated, a driver assembly causes the second and third links to move in tandem generally parallel to the longitudinally axis of the instrument in opposite directions. Consequently, the end effector rotates in the same direction as that of the actuation lever from an unarticulated position to an articulated position.

The document US 2002/072759 A1 discloses a surgical clip having a first undeployed and a second deployed shape for fastening tissue together. It comprises a connecting member having two ends, a first and a second joints, one attached to each end of said connecting member and a first and a second legs, each having a proximal and a distal end. These joints are substantially more flexible than the legs and the connecting member in one direction, but they are not conceived as articulation joints allowing the first and second legs to flex out of the common plane of the legs as the clip is advanced through an articulation instrument.

While the advances of articulating surgical instruments have helped resolve some of the problems with conventional surgical clips and instruments like clip appliers, there is still room for improvement. Because of the length, current surgical clips can not be passed through articulation instruments that have a articulation joint with a shorter length than conventional articulation instruments.

Therefore, what is desired is a surgical clip that can be completely closed and will be able to pass through surgical instruments with a shorter articulation joint.

### Summary of the Invention

In accordance with the present invention, there is provided a surgical clip having first undeployed shape, and a second deployed shape for fastening tissue together. The clip includes a connecting member having two ends and first and second flexible articulation joints one attached to each end of the connecting member. The clip further includes first and second legs each having a proximal and distal end. Wherein each of the proximal ends of the legs are attached to the articulation joints. The joints are substantially more flexible than the legs and the connecting member. The clip may be formed from said first shape to said second shape by movement of said first and second legs towards one another in a common plane. Said joints allow said first and second legs to flex out of the common plane as said clip is advanced through an articulation instrument.

### Brief Description of the Drawings

The novel features of the invention are set forth in the appended claims. The invention itself, however, together with further objects and advantages thereof, may best be understood by reference to the following description, taken in conjunction with the accompanying drawings in which:
Figure 1 is a perspective view of the surgical clip applier which can be used with the present invention.
Figure. 2 is a perspective view of the surgical clip of the present invention in an undeployed shape.
Figure 3 is a plan view of the surgical clip of the present invention in an undeployed shape.
Figure 4 is a top view of the surgical clip of the present invention in an undeployed shape.
Figure 5 is a cross sectional view of the distal end of the surgical clip shown in figure 2.
Figure 6 is a plan view of the surgical clip shown in figure 2, but showing the staple in its deployed shape.
Figure 7 is a top view illustrating the radius of curvature that prior art surgical clips can be passed through.
Figure 8 is a top view illustrating the radius of curvature that the surgical clip of the present invention can be passed through.

### Detailed Description of the Invention

Reference numerals are used in this description to designate the various components and elements of the instrument of this invention. Identical reference numerals designated in the various drawings refer to the identical element or component of the surgical penetration instrument. As used in this description, "proximal" or "proximally" refers to that portion of the instrument, component, or element which extends toward the user. Conversely, "distal" or "distally" refers to that portion of the instrument, component, or element which extends away from the user.

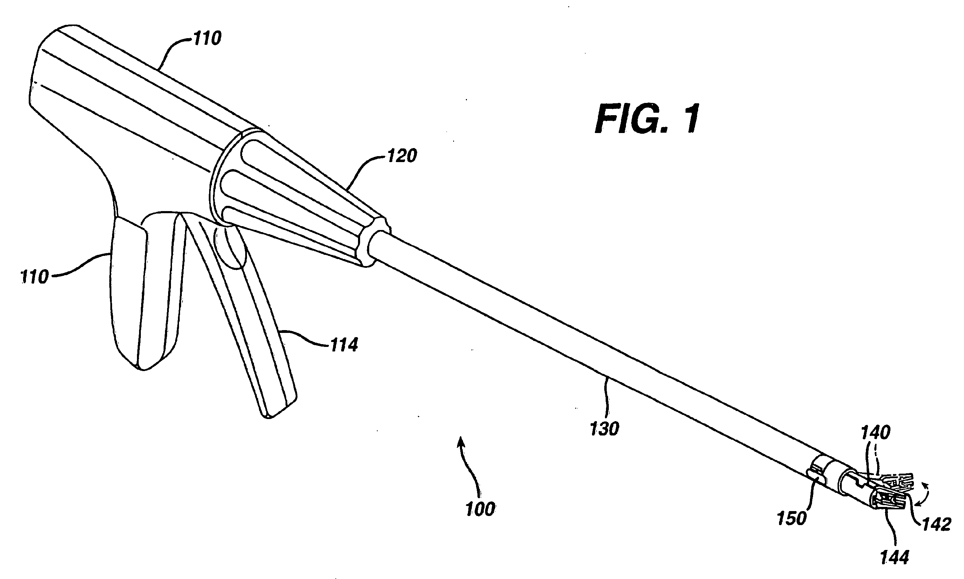

Referring now to the drawings wherein like numerals indicate the same elements throughout the views, there is shown in Figure 1 a surgical clip applier 100 designed to be used with the present invention. Clip applier 100 may be of the kind described in U.S. Patent 5,447,513 issued to Davidson et al. Clip applier 100 comprises of a handle portion 110, rotating means 120, a shaft portion 130, an anvil portion 140, and an articulation elbow 150. Anvil portion 140 further includes first jaw 142 and second jaw 144. In the handle portion 110 there may be a firing trigger 114. The firing trigger 114 causes first jaw 142 and second jaw 144 to close. The closing of first jaw 142 and second jaw 144 by firing trigger 114 causes clips to form and deploy from clip applier 100 around the tissue positioned therebetween.

As will be appreciated by those skilled in the art, the below described surgical clip has equal application for use in open clip appliers, such as those described in U.S. Patent 4,520,817 issued to Green on June 4, 1985. In addition, as used herein clip refers to any type of substantially rigid and deformable surgical fastener. Consequently, as will be appreciated by those skilled in the art, the below described clip has equal application for use in a surgical stapler, such as the one described in U.S. Patent 5,673,840 issued to Schulze et al on October 7, 1997.

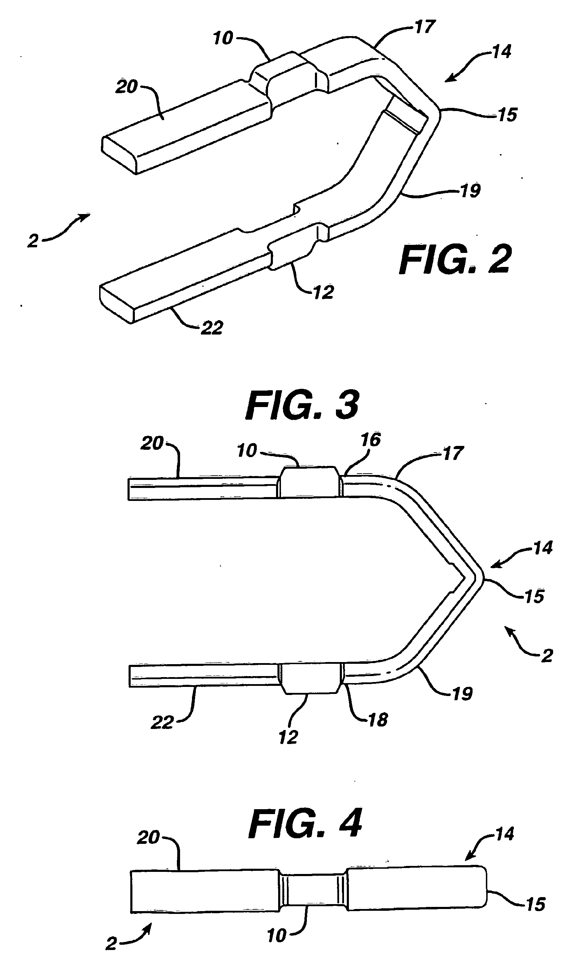

Referring now to Figures 2-4, there is shown a surgical clip 2 made in accordance with the present invention, and designed to be loaded into jaws of the type described above as item 142 and 144. As will be discussed below, clip 2 has a first undeployed shape, and a second deployed shape. Figures 2-3 depicts clip 2 in its first undeployed shape. Clip 2 includes first articulation joint 10 and second articulation joint 12 extending along the longitudinal axis of the clip 2 which are flexible and substantially less rigid than many other parts of the clip. As described below, articulating joints 10 and 12 can be given their flexibility by being formed from an elastic material such as Nitinol. However, many other constructions are possible, such as forming the joints out of hinges, springs, etc.

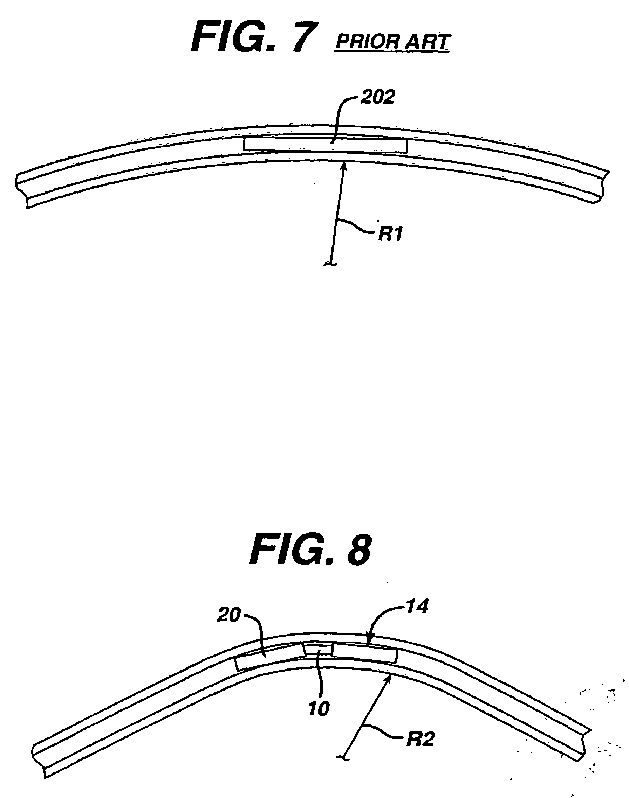

First articulation joint 10 and second articulation joint 12 generally have elongated rectangular cross-sections and may be integrally attached to clip 2. First articulation joint 10 and second articulation joint 12 have a connecting member 14 therebetween. Connecting member 14, which may be generally V-shaped, comprises of apex 15, knees 17 and 19, first end 16, and second end 18. First end 16 may be integrally attached to the proximal end of first articulation joint 10. Second end 18 may be integrally attached to the proximal end of second articulation joint 12. Clip 2 includes first elongated leg 20 extending longitudinally therefrom. First elongated leg 20 may be generally straight having a distal end and a proximal end. The proximal end of first elongated leg 20 may be integrally attached to the distal end of first articulation joint 10. Clip 2 further comprises second elongated leg 22 extending longitudinally therefrom. Second elongated leg 22 may be generally straight having a distal end and a proximal end. The proximal end of second elongated leg 22 may be integrally attached to the distal end of second articulation joint 12. As illustrated in Figure 8, first articulation joint 10 and second articulation joint 12 (not shown) can be bent laterally outward. Bending articulation joints 10 and 12 causes the effective length of the clip 2 to be roughly cut in half. Basically, by bending the articulation joints, the clip may be made of roughly two equal lengths half of the entire length of the clip.

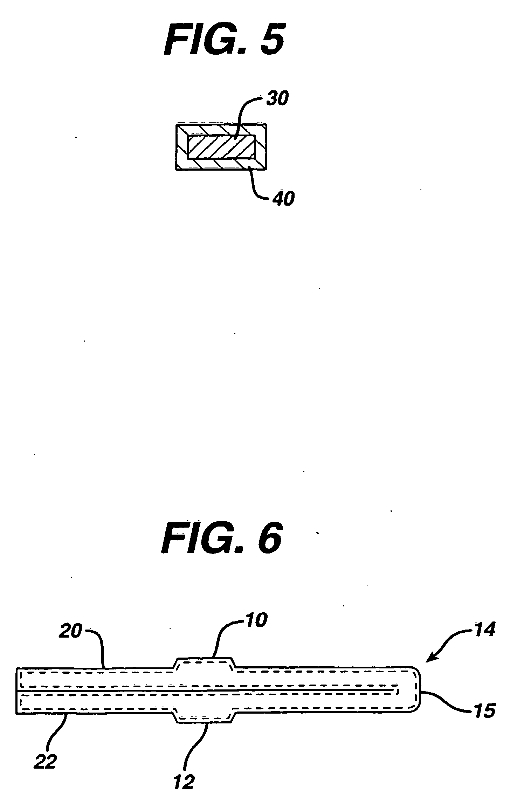

One of many possible material constructions of clip 2 can best be described by referring to Figure 5. As seen from the drawing, at least the legs, if not the entire clip, may be formed from 2 coextensive layers of material 30 and 40 joined together. As will be discussed in greater detail below, the first layer of material, or core, 30 may be made from a superelastic alloy having a relaxed configuration substantially in the clip's second shape. The second layer of material 40, or shell, may be made from a linear elastic material having a relaxed configuration substantially in the clip's first shape. The second layer of material 40 has sufficient rigidity to keep the first layer in the first shape prior to the clip being deployed.

For purposes of this invention, the first and second layers of material may be interchangeable. For example the first inner layer 30, or core, could be made from the linear elastic material, while the second outer layer 40, or shell may be constructed from a superelastic material. Moreover, it is not necessary that the layers have rectangular cross-sections, but could take on any desired shape. In addition, it is not necessary that the cross section of the clip have the core/shell configuration. The layers could be juxtaposed and coextensive with each other, or have any other desired configuration.

The first layer 30 of material may be preferably made from a superelastic or pseudoelastic alloy. One such type of material is commonly referred to as Nitinol. The nature of the superelastic transformations of shape memory alloys is discussed in "Engineering Aspects of Shape Memory Alloys", T W Duerig et al, on page 370, Butterworth-Heinemann (1990). A principal characteristic of shape memory alloys involves an initial increase in strain, approximately linearly with stress. This behavior is reversible, and corresponds to conventional elastic deformation. Subsequent increases in strain are accompanied by little or no increase in stress, over a limited range of strain to the end of the "loading plateau". The loading plateau stress is defined by the inflection point on the stress/strain graph. Subsequent increases in strain are accompanied by increases in stress. On unloading, there is a decline in stress with reducing strain to the start of the "unloading plateau" evidenced by the existence of an inflection point along which stress changes little with reducing strain. At the end of the unloading plateau, stress reduces with reducing strain. The unloading plateau stress is also defined by the inflection point on the stress/strain graph. Any residual strain after unloading to zero stress is the permanent set of the sample. Characteristics of this deformation, the loading plateau, the unloading plateau, the elastic modulus, the plateau length and the permanent set (defined with respect to a specific total deformation) are established, and are defined in, for example, "Engineering Aspects of Shape Memory Alloys", on page 376.

Non-linear superelastic properties can be introduced in a shape memory alloy by a process which involves cold working the alloy for example by a process that involves pressing, swaging or drawing. The superelastic properties are employed by the staple in its change of configuration between its first or undeployed/restrained shape, and its second or deployed/relaxed shape. An appropriate treatment can involve a combination of cold working (for example by swaging, drawing or, in particular by mandrel expansion) and heat treatment at a temperature that is less than the recrystallisation temperature of the alloy while the stent is constrained in the configuration resulting from the cold work. A plurality of the cold work and heat treatment steps can be used. The clip leg can then be deformed towards undeployed shape, the deformation being recoverable, substantially elastically. In this way, deformations of up to 8% strain can be imparted and recovered substantially elastically. The alloy for the first layer 30 is preferably manufactured such that it exhibits superelastic properties at body temperature.

Preferable Nitinol or Ni-Ti binary alloys for the first layer of material have a nickel content of at least about 50 atomic percent (hereinafter at. %), preferably at least about 50.5 at. %. The nickel content will usually be less than about 54 at. %, preferably less than about 52 at. %. As will be appreciated by those skilled in the art, the first layer can be made from other Ni-Ti based alloys, including alloys with ternary and quaternary additions. Examples of elements that can be incorporated in the alloy include Fe, Co, Cr, Al, Cu and V. Added elements can be present in amounts up to about 10 at. %, preferably up to about 5 at. %.

The second layer of material 40 may be preferably made from a linear elastic material, such as iron, stainless steel or titanium linear elastic nitinol. The second layer could also be made from a material which would impart radiopaque qualities to the clip so it could be seen better under x-ray. The yield strength of the second layer of material may be set to be modestly higher than the recovery strength of the first layer of material except at first articulation joint 10 and second articulation joint 12. At the articulation joints 10 and 12, the yield strength of the first layer of material may be set to be modestly higher than the recover strength of the second layer of material to enable the clip to be self straightening after it is passed through a curved track of an articulating elbow into a straight track before reaching the jaws. Alternately, the yield strength of the second layer of material may be set to be modestly higher than the recovery strength of the first layer of material throughout the entire clip including articulation joints 10 and 12.

For example purposes, the manufacturing of the clip will now be described, wherein the second layer 40 comprises iron. The clip can be initially manufactured by co-drawing a rectangular covering of iron around a Nitinol rectangular core until you have a rectangular shape having the cross-section shown in Figure 5. That is the rectangular shape can be formed by sliding a length of Nitinol rectangular core inside a length of iron rectangular covering and then drawing the two together until the desired shape and size of the rectangular clip is produced. The articulation joints could be formed in the same manner just increasing the height of the rectangular core and covering locally at these segments. The shape and size of the Nitinol core, the wall thickness of the iron cover, and the level of work hardening in the cover can be varied to create clips with varying degrees of biased-properties.

The rectangular clip can then be cut into a desired clip size length segments. Thereafter the segment is cooled so that the Nitinol is substantially martensitic, and then the segment is deformed into its desired second/deployed shape, shown in Figure 6. The segment is then heat treated to shape set the Nitinol and partially stress relieve the Titanium. After the Nitinol in the clip had been shape-set, the clip could be opened and bent to the geometry depicted in Figures 2-3 to form clip 2 which will then be loaded into and used in conventional clip appliers.

The clip 2 combines shape-memory and linear-elastic materials such that the clip has some of the properties of shape-memory materials and some of the properties of linear-elastic materials. When deploying the clip, such as ejecting it from a pair of closed jaws, the sum of applied stresses and internally generated shape-memory recovery stresses exceed the yield strength of the linear-elastic material such that the clip will deform. When the loads are applied in such a fashion that they aid the shape-set material recovery stresses and the external load required to cause deformation will be lower than if the forces were applied to the linear-elastic portion of the clip alone. The apex 15 and knees 17 and 19 of the connecting member 14 may be optimized to flex preferentially in the vertical plane, with the strength of the knees 17 and 19 balanced to minimize crushing forces while remaining slightly stronger than the apex 15 at resisting opening forces at the distal tips of the clip. In contrast, first articulation joint 10 and second articulation joint 12 may be optimized to flex preferentially in the lateral, or horizontal plane, while having increased strength and stiffness in the vertical plane. The goal of this is for the articulation joints to flex before other portions of the clip body during transport through the curved clip track, but resist flexing during the clip crushing process.

When the articulated clip emerges from the curved clip track in articulation elbow 150 into a straight section before entering the instrument jaws, one of two things will take place. If the clip has been made such that the yield strength of the first layer of material is set to be modestly higher than the recover strength of the second layer of material at the articulation joints 10 and 12, the clip should primarily straighten itself. If the clip has been made such that the yield strength of the second layer of material is set to be modestly higher than the recover strength of the first layer of material at the articulation joints 10 and 12, means will be needed to straighten the clip. A short straight portion of clip track in clip applier 100 alone may be adequate to straighten the clip well enough to be fed and used. If needed, additional guide tracks can be used to assist in guiding the clip into the jaws.

As the clip is deployed, the clip would begin deforming and assuming the desired closed "U" shape at much lower loads than a conventional clip. This means that even at early stages of clip formation, the tips of the clip would close together and eventually close themselves as shown in Figure 6. Since the length of a clip dictates the radius of curvature of the articulation elbow a clip can pass through, clip 2 having a bend at the articulation joints 10 and 12 can be passed through clip appliers or surgical instruments that have an articulation elbow with a smaller radius of curvature R2, as shown in Figure 8. In contrast, a conventional clip, which has an effective length greater than clip 2, can not be passed through clip appliers or surgical instruments with shorter articulation elbows and thus a smaller radius of curvature. In fact, referring to Figure 7, the radius of curvature R1 needed for a conventional clip 202 is larger than the clip of the present invention shown as R2 in Figure 8. The above mentioned clip and its associated geometry reduce these drawbacks.

Radial forming forces would remain lower for the above described clip throughout the forming process providing the clips were originally formed and shape-set at a formed height slightly less than the clip applier could stroke to form them, even in thin tissue. This fundamental reduction in clip forming forces would have a ripple effect throughout the instrument because the tendency to force the jaws apart would be reduced.

The properties of the above mentioned clip could cause a manufacturer to decrease the length of the articulation elbow of most conventional clip appliers that feed that comprise of multiple clips. In addition, it could also cause a manufacturer to use insert multiple clips and a feeding system in current clip appliers and surgical instruments with a shorter articulation elbow that allow for only a single clip to be loaded at a time. In articulating clip appliers clips can't be fed through the articulating clip appliers, clips can't be fed through the articulation joint while it's articulated this invention would support feeding while articulated.

While preferred embodiments of the present invention have been shown and described herein, it will be obvious to those skilled in the art that such embodiments are provided by way of example only. Accordingly, it is intended that the invention be limited only by the scope of the appended claims.

## Claims

1. A surgical clip (2) having a first non-deployed shape, and a second, deployed shape for fastening tissue together, said clip comprising:
a connecting member (14) having two ends (16, 18);
first and second flexible articulation joints, (10, 12) one attached to each end of said connecting member;
first and second legs (20, 22), each having a proximal and a distal end, wherein each of said proximal ends of said legs are attached to said articulation joints;
wherein said clip (2) may be formed from said first shape to said second shape by movement of said first and second legs (20, 22) towards one another in a common plane; and
said articulation joints (10, 12) are substantially more flexible than said legs and said connecting member, thereby allowing said first and second legs to flex out of the common plane as said clip is advanced through an articulation instrument.

2. The surgical clip according to claim 1, wherein said articulation joints are elastic.

3. The surgical clip according to claim 2 wherein said legs comprise a linear elastic material.

4. The surgical clip according to claim 1, wherein said connecting member comprises a V shape apex.

5. The surgical clip of any one of claims 1-4 in which the said articulation joints allow said first and second legs to flex more easily out of said common plane than within said common plane.

6. A surgical clip according to claim 1, wherein said surgical clip comprises a first layer of material comprising a superelastic alloy, wherein said first layer has a relaxed configuration substantially in said second shape, and wherein said legs and said connecting member further comprise a second layer of material joined to said first layer, wherein said second layer comprises a linear elastic material having a relaxed configuration substantially in said first shape and having sufficient rigidity to keep said first layer and said connecting member and said legs in said first shape prior to said clip being deployed.

7. The surgical clip according to claim 6, wherein said superelastic alloy comprises a nickel titanium alloy.

8. The surgical clip according to claim 6, wherein said superelastic alloy has an Af temperature below body temperature.

9. The surgical clip according to claim 6, wherein said superelastic alloy has an Af temperature below 20 degrees Centigrade.

## Patentansprüche

1. Chirurgische Klammer (2) mit einem ersten, uneingesetzten Zustand und einem zweiten, eingesetzten Zustand zum Festhalten von Gewebe aneinander, welche Klammer umfaßt:
ein Verbindungsteil (14) mit zwei Enden (16, 18);
eine erste flexible Verbindung (10) und eine zweite flexible Verbindung (12), von denen eine an jedem Ende des Verbindungsteils angebracht ist;
ein erstes Bein (20) und ein zweites Bein (22) mit jeweils einem proximalen und einem distales Ende, wobei jedes proximale Ende der Beine an den Verbindungen angebracht ist;
wobei die Klammer (2) von dem ersten Zustand in den zweiten Zustand verformbar ist, indem das erste Bein (20) und das zweite Bein (22) in eine gemeinsame Ebene aufeinander zu bewegt werden; und
wobei die Verbindungen (10, 12) im wesentlichen flexibler als die Beine und das Verbindungsteil sind, wodurch sich das erste und das zweite Bein aus der gemeinsamen Ebene biegen kann, wenn die Klammer durch ein Verbindungsgerät hindurch vorgeschoben wird.

2. Chirurgische Klammer nach Anspruch 1, bei der die Verbindungen elastisch sind.

3. Chirurgische Klammer nach Anspruch 2, bei der die Beine ein lineares elastisches Material aufweisen.

4. Chirurgische Klammer nach Anspruch 1, bei der das Verbindungsteil eine V-förmige Spitze aufweist.

5. Chirurgische Klammer nach einem der Ansprüche 1 bis 4, bei der die Verbindungen ein leichteres Herausbiegen des ersten und zweiten Beins aus der gemeinsamen Ebene als ein Biegen innerhalb der gemeinsamen Ebene zulassen.

6. Chirurgische Klammer nach Anspruch 1, die eine erste Materialschicht umfaßt, die eine superelastische Legierung aufweist, im wesentlichen im zweiten Zustand entspannt ist, wobei die Beine und das Verbindungsteil zudem eine zweite Materialschicht umfassen, die mit der ersten Materialschicht verbunden ist, wobei die zweite Materialschicht ein lineares elastisches Material aufweist, das im wesentlichen im ersten Zustand entspannt ist und eine ausreichende Festigkeit aufweist, um die erste Materialschicht und das Verbindungsteil und die Beine in dem ersten Zustand zu halten, bis die Klammer eingesetzt ist.

7. Chirurgische Klammer nach Anspruch 6, bei der die superelastische Legierung eine Nickel-Titan-Legierung ist.

8. Chirurgische Klammer nach Anspruch 6, bei der die superelastische Legierung eine Af-Temperatur unterhalb einer Körpertemperatur aufweist.

9. Chirurgische Klammer nach Anspruch 6, bei der die superelastische Legierung eine Af-Temperatur von unter 20°C aufweist.

## Revendications

1. Clip chirurgical (2) ayant une première forme non déployée, et une seconde forme déployée pour agrafer du tissu ensemble, ledit clip comprenant :
un élément de raccordement (14) ayant deux extrémités (16, 18),
des première et seconde charnières d'articulation (10, 12), chacune attachée à chaque extrémité dudit élément de raccordement ;
des premières et secondes pattes (20, 22), ayant chacune une extrémité proximale et une extrémité distale, dans lesquelles chacune desdites extrémités proximales desdites pattes (20, 22) sont reliées aux dites charnières d'articulation ;
dans lequel ledit clip (2) peut passer de ladite première forme à ladite seconde forme par un mouvement desdites première et seconde pattes (20, 22) en direction l'une de l'autre dans un plan commun ; et
lesdites charnières d'articulation (10, 12) sont essentiellement plus flexibles que lesdites pattes et ledit élément de raccordement, permettant ainsi aux dites première et seconde pattes de fléchir hors du plan commun tandis que ledit clip est avancé à travers un instrument d'articulation.

2. Clip chirurgical selon la revendication 1, dans lequel lesdites charnières d'articulation (1) sont élastiques.

3. Clip chirurgical selon la revendication 2, dans lequel lesdites pattes comprennent un matériau élastique linéaire.

4. Clip chirurgical selon la revendication 1, dans lequel ledit élément de raccordement comprend un sommet en forme de V.

5. Clip chirurgical selon l'une quelconque des revendications 1 à 4, dans lequel lesdites charnières d'articulation (1) permettent aux dites première et seconde pattes de fléchir plus aisément en dehors du plan commun que dans ledit plan commun.

6. Clip chirurgical selon la revendication 1, dans lequel ledit clip chirurgical comprend une première couche de matériau comprenant un alliage super élastique, dans lequel ladite première couche présente une configuration détendue essentiellement dans ladite seconde forme, et dans lequel lesdites pattes et ledit élément de raccordement comprennent en outre une seconde couche de matériau reliée à ladite première couche, dans lequel ladite seconde couche comprend un matériau élastique linéaire ayant une configuration détendue essentiellement dans ladite première forme et ayant une rigidité suffisante pour maintenir ladite première couche et ledit élément de raccordement et lesdites pattes dans ladite première forme avant le déploiement dudit clip.

7. Clip chirurgical selon la revendication 6, dans lequel ledit alliage super élastique comprend un alliage de nickel-titane.

8. Clip chirurgical selon la revendication 6, dans lequel ledit alliage super élastique a une température Af inférieure à la température corporelle.

9. Clip chirurgical selon la revendication 6, dans lequel ledit alliage super élastique a une température Af inférieure à 20 degrés Centrigrade.
